**Europäisches Patentamt**

**European Patent Office**

**Office européen des brevets**

(11) Veröffentlichungsnummer: **0 174 669**

**A2**

(12) # EUROPÄISCHE PATENTANMELDUNG

(21) Anmeldenummer: **85112101.2**

(22) Anmeldetag: **26.03.82**

(51) Int. Cl.⁴: **A 61 K 39/385**
**A 61 K 39/29, C 12 N 15/00**

(30) Priorität: **28.03.81 DE 3112338**

(43) Veröffentlichungstag der Anmeldung:
**19.03.86 Patentblatt 86/12**

(84) Benannte Vertragsstaaten:
**AT BE CH DE FR GB IT LI LU NL SE**

(60) Veröffentlichungsnummer der früheren
Anmeldung nach Art. 76 EPÜ: **0 061 740**

(71) Anmelder: **Von der Helm, Klaus**
**Glatzerstrasse 33**
**D-8034 Gemering(DE)**

(71) Anmelder: **Winnacker, Ernst-L.**
**Elvirastrasse 4**
**D-8000 München 19(DE)**

(71) Anmelder: **Deinhardt, Friedrich**
**Hermine-Bland-Strasse 9**
**D-8000 München 90(DE)**

(72) Erfinder: **Von der Helm, Klaus**
**Glatzerstrasse 33**
**D-8034 Gemering(DE)**

(72) Erfinder: **Winnacker, Ernst-L.**
**Elvirastrasse 4**
**D-8000 München 19(DE)**

(72) Erfinder: **Deinhardt, Friedrich**
**Hermine-Bland-Strasse 9**
**D-8000 München 90(DE)**

(74) Vertreter: **von Kreisler, Alek, Dipl.-Chem.**
**Deichmannhaus am Hauptbahnhof**
**D-5000 Köln 1(DE)**

(54) **Verfahren zur Herstellung einer Hybrid-DNA, die das komplementäre Genom des Hepatitis-A-Virus enthält, diese Hybrid-DNA und den sie enthaltenden Plasmidvektor, von der Hybrid-DNA exprimierte Virus-spezifische Proteine und diese enthaltende und damit hergestellte Impfstoffe.**

(57) Die Erfindung betrifft ein Verfahren zur Herstellung einer Hybrid-DNA, die das für das Heptatitis-A-Virus (HAV) charakteristische komplementäre Genom enthält, die so hergestellte Hybrid-DNA, den sie enthaltenden Plasmidvektor, die in Bakterienklonen exprimierten Proteine oder Hybridproteine, sowie die Verwendung dieser Habrid-DNA zum Nachweis von Hepatitis-A-Virus in einer biologischen Probe, und Impfstoffe, die diese Proteine oder Hybridproteine enrhalten.

EP 0 174 669 A2

VON KREISLER   SCHÖNWALD   EISHOLD   FUES
VON KREISLER   KELLER   SELTING   WERNER

PATENTANWÄLTE

Dr.-Ing. von Kreisler † 1973
Dr.-Ing. K. Schönwald, Köln
Dr.-Ing. K. W. Eishold, Bad Soden
Dr. J. F. Fues, Köln
Dipl.-Chem. Alek von Kreisler, Köln
Dipl.-Chem. Carola Keller, Köln
Dipl.-Ing. G. Selting, Köln
Dr. H.-K. Werner, Köln

DEICHMANNHAUS AM HAUPTBAHNHOF
**D-5000 KÖLN 1**

AvK/IM

0174669

Verfahren zur Herstellung einer Hybrid-DNA, die das
komplementäre Genom des Hepatitis-A-Virus enthält,
diese Hybrid-DNA und den sie enthaltenden Plasmidvektor,
von der Hybrid-DNA exprimierte virusspezifische Proteine und diese
enthaltende und damit hergestellte Impfstoffe

Die Erfindung betrifft ein Verfahren zur Herstellung einer
Hybrid-DNA, die das für das Heptatitis-A-Virus (HAV)
charakteristische komplementäre Genom enthält, die so hergestellte Hybrid-DNA, den sie enthaltenden Plasmidvektor,
die in Bakterienklonen exprimierten Proteine oder Hybridproteine, sowie die Verwendung dieser Hybrid-DNA zum Nachweis von Hepatitis-A-Virus in einer biologischen Probe,
und Impfstoffe, die diese Proteine oder Hybridproteine
enthalten, daraus hergestellt sind oder anhand der Nucleotidsequenz
der Hybrid-DNA hergestellt sind.

Hepatitis A (HA) bleibt eine Krankheit, die im Rahmen der
öffentlichen Gesundheit von großer Bedeutung ist, und ihr
Verursacher wurde erst kürzlich identifiziert. Die Arbeitsausfälle sind lang, die Krankenkosten (durch z.B. Isolierung
des Kranken) hoch. Eine passive, besser noch allgemeine aktive
Impfung wäre von großem gesundheitspolitischem und volkswirtschaftlichem Nutzen. Dazu müsste der Erreger dieser
Krankheit, das Hepatitis-A-Virus (HAV) in großer Menge zur
Verfügung stehen um a) wissenschaftliche Studien zu treiben,
b) das virale Antigen zur Immunisierung benutzen zu können.
Das ist augenblicklich nicht der Fall. Virusmaterial steht noch nicht einmal in solcher Menge zur Verfügung, daß eine
ausreichende biochemisch-virologische Untersuchung des Viruspartikels durchgeführt werden kann.

Hepatitis A wurde 1967 - 1970 erstmals auf nicht-menschliche Primaten übertragen (ein Überblick wurde von
Deinhardt, F., 1976, in "Advances in Virus Research,
Vol. 20, Herausgeber F.B. Bang (Academic Press, New
York), S. 113, gegeben), und Viruspartikel wurden später
im Stuhl von in der akuten Phase befindlichen Patienten
durch Immunelektronenmikroskopie von Feinstone et al
1973 identifiziert (Feinstone, S.M., A.Z. Kapikian und
R.H. Purcell, Science 182 (1973) 1026).

Die biophysikalischen und biochemischen Eigenschaften des Hepatitis-A-
Virus (HAV) wurden von Provost et al (P.H. Provost,
B.S. Wolanski, W.J. Miller, O.L. Ittensohn, W.J. McAleer
und M.R. Hilleman, Proc. Soc. Exp.Biol. Med. 148 (1975)
532), Siegl und Frösner (G. Siegl und G.G. Frösner,
J. Virol. 26 (1978) 40 und 48), Coulepis et al (A.G.
Coulepis, S.A. Locarnini, A.A. Ferris, N.I. Lehmann und
I.D. Gust, Intervirology 10 (1978) 24 und A.G. Coulepis,
S.A. Locarnini und I.D. Gust, J. Virol. 35 (1980) 572),
Bradley et al (D.W. Bradley, C.L. Hornbeck, E.H. Cook
und J.E. Maynard, J. Virol. 22 (1978) 228 und D.W.Bradley,
H.A. Fields, K.A. McCaustland, E.H. Cook, C.R. Gravelle
und J.E. Maynard, J. Med.Virol. 2 (1978) 175), Feinstone
et al  (S.M. Feinstone, Y. Moritsugu, J.W.K. Shih,
J.L. Gerin und R.H. Purcell in "Viral Hepatitis", herausgegeben von G.N. Vyas, S.N. Cohen und R. Schmidt (Abacus
Press, Turnbridge Wells) 1979, S. 41),  Maynard und
Bradley (J.E. Maynard und D.W. Bradley (1980) in "Virus
and the Liver", herausgegeben von L. Bianchi, W. Gerock,
K. Sickinger und A. Stadler (MTP Press Ltd., Lancaster),
S. 9) und Tratschin et al (J.D. Tratschin, G. Siegl,
G.G. Frösner und F. Deinhard, 1981, J. Virol., im Druck) bestimmt, und es be̶ ̶ht nun allgemeine Übereinstimmung,
daß HAV ein Picornavisus mit den meisten Charakteristiken
eines Enterovirus ist.

Das Genom von HAV ist eine einsträngige lineare RNA
(wahrscheinlich mit Poly-A-Regionen am 3'-Ende) mit einer
Größe von 2,25 bis 2,8 x $10^6$ Dalton und einem Sedimentationskoeffizienten von 32,5 bis 33 S im Vergleich zu Poliovirus, das etwa 6500 Nucleotiden entspricht. Die Genkarte
von HAV und die Expression der Gene sind unbekannt. Über
die Struktur der Genprodukte der HAV-Proteine und ihre
Antigenizität ist wenig bekannt.

Obwohl HAV kürzlich in Zellkulturen gezüchtet worden ist
(P.H. Provost und M.R. Hilleman, Proc.Soc.Exp.Biol.Med.
160 (1979) 231; G.G. Frösner, F. Deinhardt, R. Scheid,
V. Gauss-Müller, N. Holmes, V. Messelberger, G. Siegl und
J.J. Alexander, Infection 7 (1979) 303; B. Flehmig,
Münch. med. Wschr. 119 (1977) 825 und F. Deinhardt, R. Scheid,
V. Gauss-Müller, G.G. Frösner und G. Siegl, Progr. Med.Virol.
27 (1981) im Druck, zeigen infizierte Zellen keinen cytophatischen Effekt (CPE), d.h. sie werden durch die Virusinfektion nicht abgetötet und Viruspartikel werden nicht
freigegeben. Jedoch ist der Wachstumszeitraum in der Gewebekultur ungewöhnlich lang, die Ausbeute relativ gering
und stark durch die Tatsache behindert, daß das Virus
nicht von den Kulturzellen freigesetzt wird, sondern durch
künstliche Zerstörung dieser Zellen gewonnen werden muss,
wobei die Gefahr der Kontamination von zellulärem Material
außergewöhnlich hoch ist. Die Reinigung von HAV aus solchen
Kulturen ist kompliziert, weil infizierte Zellen lysiert
und HAV von den Zelltrümmern abgetrennt werden muss.

Es besteht daher ein Bedürfnis, in ausreichender Menge
sauberes Virusmaterial des HAV zu erhalten, um HAV-Antigene
darzustellen, die eine immunisierende Wirkung gegen HAV
aufweisen.

Die Erfindung stellt sich daher die Aufgabe, ein Verfahren
zur Herstellung einer Hybrid-DNA, die das für das Hepatitis-
A-Virus charakteristische komplementäre Genom enthält, den
die Hybrid-DNA enthaltenden Plasmidvektor, HAV-spezifische
Proteine und Hybridproteine, die HAV-spezifische Antigenizität tragen, in ausreichender Menge einfach zur Verfügung zu
stellen.
Diese und andere Aufgaben, die sich aus der nachfolgenden
Beschreibung ergeben, werden durch die Erfindung gelöst.

Dementsprechend betrifft die Erfindung ein Verfahren zur
Herstellung einer Hybrid-DNA, die das für das Hepatitis-A-
Virus charakteristische komplementäre Genom enthält, das
dadurch gekennzeichnet ist, daß man doppelsträngige DNA,
die durch reverse Transkription der viralen RNA des
Hepatitis-A-Virus hergestellt ist, in einem Bakterienplasmid
kloniert.

Die Erfindung betrifft außerdem doppelsträngige Hybrid-DNA-
Moleküle, von denen bestimmte Restriktions-Fragmente komplementär zu der RNA des Hepatitis-A-Virus sind.

Weiterhin betrifft die Erfindung Plasmidvektoren und transformierte Bakterien, die diese Hybrid-DNA-Moleküle enthalten.

Die Erfindung betrifft weiterhin ein Verfahren zur Herstellung von Proteinen, die ein Proteinfragment mit immunisierender Wirkung gegen Hepatitis-A-Virus enthalten, diese
Proteine oder Hybridproteine, sowie diese enthaltende Impfstoffe.
Schließlich betrifft die Erfindung die Verwendung der Hybrid-
DNA zum Nachweis der Anwesenheit von Hepatitis-A-Virus-spezifischen RNA-Sequenzen.

Gemäss der Erfindung wird das HAV-Genom in Form einer
komplementär DNA (= cDNA) in ein Bakterienplasmid kloniert
(eingebaut), die Bakterienklone werden gezüchtet, das Genom
wird repliziert und einzelne Gene werden exprimiert, so
daß vollständige oder teilweise Produktion von viralen
Proteinen erfolgt, die HAV-spezifische Antigenizität aufweisen. Dabei wird wie folgt verfahren:

Vom Stuhl entnommenes Hepatitis-A-Virus (HAV) wurde stark
gereinigt. Die genomische RNA wurde zu cDNA mittels der AMVreversen Transcriptase transkribiert, und diese DNA wurde
dann in den Pst I-Ort des Plasmid pBR 322 kloniert, und
Klone wurden in Gegenwart von Tetracyclin selektiert.
Die meisten Klone enthielten Einfügungen im Plasmid, die
mit HAV-spezifischer RNA hybridisieren, die von HAV-infizierten Zellkulturen isoliert worden war, die von einem
menschlichen hepatocellulären Karzinom stammten. Einige
Klone zeigten Expression unterschiedlicher Mengen
viraler Antigene, die verwendet werden kann zur Immunisierung gegen HAV.

Der Vorteil dieser Methode ist die fast unbegrenzt herstellbare Menge von viralem Material, die als Bakteriensuspension
in großindustriellem Maßstab erfolgen kann. Weiter liegt
der Vorteil in der Möglichkeit, gezielt nur dasjenige Virusmaterial, d.h. die viralen Antigenproteine, herstellen zu
können, die zum Immunitätsprozess benötigt werden, nicht
aber anderes Virusmaterial, das als Ballast kostenaufwendig
ist. Vor allem wird bei dieser Methode des Klonierens vermieden, infektiöses Material (Virusgenom) mit herzustellen,
das unter großem Aufwand wieder entfernt oder weggereinigt
werden müsste. Die genetische Information für die viralen
Antigene bleibt in dem Bakterium verankert, die Bakterien
produzieren nur das Immunmaterial.

Die neue Hybrid-DNA gemäss der Erfindung, die erfindungsgemäss hergestellt wird, kann von Vorteil in verschiedenen
Anwendungsgebieten verwendet werden. Beispielsweise kann
man damit routinemässig diagnostisch das Vorhandensein von HAV in
klinischem Material nachweisen. Beispielsweise können dazu
diese Hybrid-DNA-Moleküle verwendet werden, die nach bekannten Verfahren radioaktiv markiert worden sind, oder es können
die exprimierten Proteine dazu verwendet werden.
Die erfindungsgemässen neuen Plasmidvektoren, die nach dem
Verfahren der Erfindung erhalten worden sind und deren Genome
die erfindungsgemässen Hybrid-DNA enthalten, können dazu
verwendet werden, um in Bakterien exprimiert zu werden,
wodurch die Herstellung eines HAV-spezifischen Proteins
in Form eines freien oder Hybridproteins möglich wird. Das
(Hybrid)Protein enthält das HAV-Antigen und kann zur
Herstellung von Impfstoffen gegen Hepatitis-A eingesetzt
werden.
Die Erfindung wird nachstehend weiter anhand der Abbildungen erläutert.

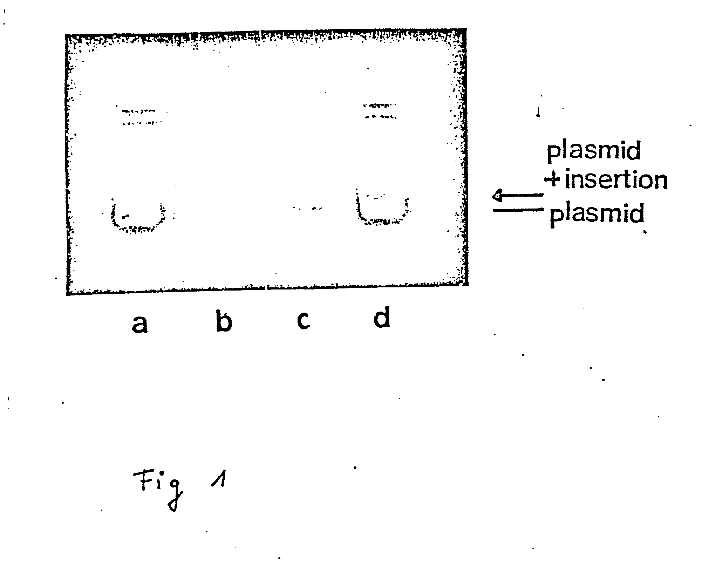

Fig. 1: Größe der eingefügten DNA

Einzelne Bakterienklone wurden gezüchtet und Plasmide
hergestellt. Die Größe der Plasmide wurde durch Elektrophorese auf 1%igem Agarosegel ermittelt und Banden durch
Ethidiumbromid in UV-Licht sichtbar gemacht. Die Aufnahmen
2 und 3 zeigen zwei typische Plasmide mit DNA-Einfügung,
und die Aufnahmen 1 und 4 stellen das Bezugsplasmid
pBR 322 ohne Einfügung dar.

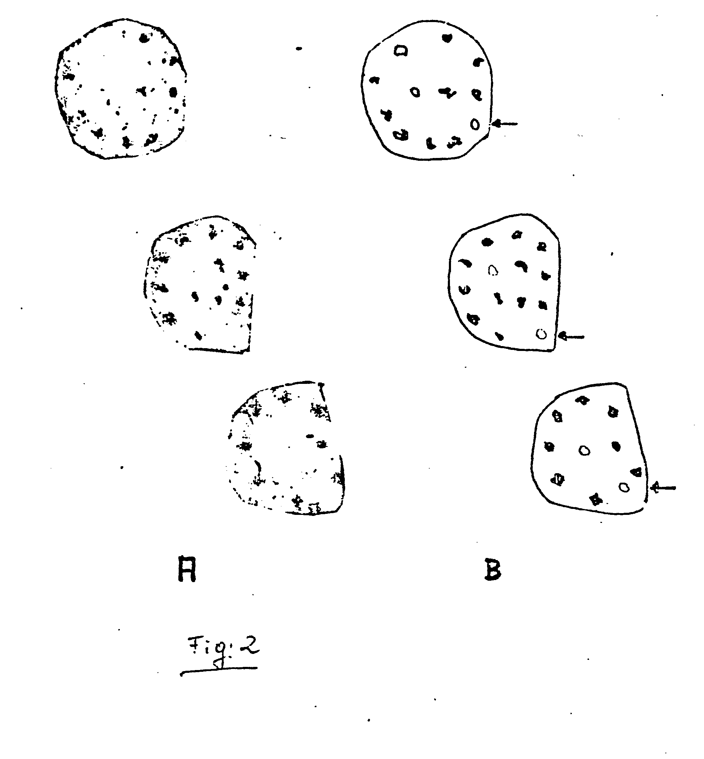

Fig. 2: Hybridisierung von Plasmid-Einfügungen mit
$^{32}$P-markierter HAV-RNA

Klone wurden auf Nitrocellulosefiltern über Agar gemäß
Grunstein und Hogness (loc.cit.) gezüchtet.HAV-spezifische RNA, gewonnen von mit HAV infizierten PLC/PRF5-
Zellen (Alexander et al, 1978, loc.cit.) und mit $^{32}$P
markiert, wurde zu den auf Nitrocellulosefiltern gezüchteten Bakterienklonen hybridisiert. A) Autoradiogramm
von drei Filtern mit gewachsenen Klonen nach Hybridisierung
mit HAV-spezifischer RNA; B) schematische Darstellung
dieser drei Filter (die Pfeile unten rechts stellen Kontrollkolonien dar, die pBR 322-DNA ohne Einfügung enthalten); Klone ● = Hybridisierung positiv; Klone 0 = Hybridisierung negativ.

Fig. 3:
Wasserschock-Lysate verschiedener Mengen von aliquoten
Teilen einzelner Bakterienklone wurden einem HAV.-Radioimmuntest unterworfen. A) Radioimmuntests mit Kugeln,
die mit Anti-HAV-positivem menschlichen Serum umhüllt
sind; B) mit Kugeln, die mit Anti-HAV-negativem menschlichen Serum umhüllt sind.

Für die Untersuchung wurden HAV-Partikel aus einem Stuhl
hergestellt, der von einem Patienten, bei dem sich vier
Tage später typische Hepatitis A einstellte, erhalten
worden war. Die Gesamtausbeute an Partikeln entsprach
ungefähr einer Virusmasse von etwa 10 bis 20 µg. Aus den
Partikeln extrahierte RNA wurde mit reverser Transcriptase des Myeloblastosis-Virus von Vögeln (AMV) in cDNA
transkribiert, und die Analyse der cDNA durch Gel-Elektrophorese ergab eine ungefähre Ausbeute von 10 bis 50 ng DNA
von ineinheitlicher Größe mit der überwiegenden Population
bei etwa 800 bis 1000 Basenpaaren. Die DNA wurde in den
Pst I-Restriktionsort des Plasmids pBR 322 kloniert.
E. coli X 1776 wurde dann durch die Hybridplasmide transformiert, und Klone wurden in Gegenwart von Tetracyclin
selektiert. Aus einzelnen Bakterienklonen wurden Plasmide
hergestellt. Die Analyse der klonierten Plasmide ergab,
daß viele eine DNA-Einfügung (Fig. 1) im Bereich von
300 bis 800 Nucleotiden enthielten.

Um zu ermitteln, ob die DNA-Einfügung HAV-spezifische
Nucleotidsequenzen aufwies, wurden die rekombinierten
Plasmide zu HAV-spezifischer, mit $^{32}$p markierter RNA
hybridisiert. Diese RNA-Proben wurden aus einer anderen
Quelle hergestellt, nämlich aus mit HAV infizierten Zellkulturen, um falsche Ergebnisse als Folge einer Kontamination mit nicht-viraler RNA der für die Transkription verwendeten ursprünglichen, vom Stuhl gewonnenen viralen
RNA zu vermeiden. Die meisten Klone, die Einfügungen
enthielten, hybridisierten zu dieser HAV-RNA (Fig. 2).

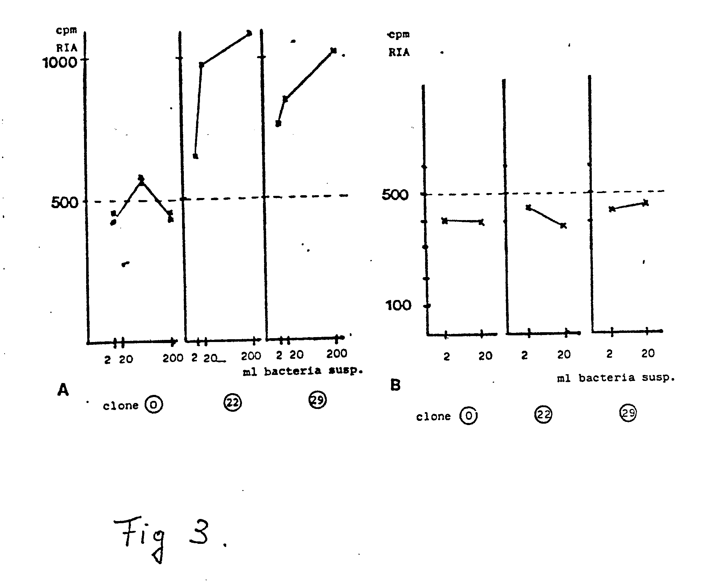

Die Bakterienklone wurden auf Expression von Antigen nach
Wasserschock-Lysis der Bakterien (Villa-Komaroff et al,
loc.cit.) untersucht, und die Schockfluids wurden in einem
Festphasen-Radioimmuntest (HAVAB von Abbott Laboratories,
North Chicago, USA) auf HAV-Antigene getestet. Zwei
Klone waren positiv, d.h. sie reagierten auf einem 2- bis
3fachen Niveau über dem negativen Hintergrund. Steigende
Mengen von Schockfluids (2,20 bzw. 200 ml) banden proportional steigende Mengen von Radioaktivität beim Radioimmuntest (Fig. 3A).

Um diese Ergebnisse zu bestätigen, wurde der Radioimmuntest unter Verwendung einer festen Phase (Kugeln) durchgeführt, die mit HAV-negativem Serum umhüllt worden waren.
Fig. 3B zeigt, daß kein Schockfluid unter diesen Bedingungen
reagierte, ein weiterer Beweis, daß HAV-spezifische Polypeptide in den Klonen 22 und 29 exprimiert sind.

Die vorhandenen Klone können nunmehr als Hybridisierungssonden und Primer für die Herstellung von weiterer cDNA unter
Verwendung von RNA von mit HAV infizierten Zellkulturen
verwendet werden.

## Reinigung von Viruspartikeln

HAV-Partikel wurden aus 25 g gefrorenem Stuhl gereinigt wie beschrieben
(G. Siegl und G.G. Frösner, J. Virol. 26 (1978) 40 und
48). Die Partikel wurden, kurz gesagt, aus dem Stuhl
entnommen, an einem vorgebildeten CsCl-Dichtegradienten
(Dichte 1,25-1,50 g/cm$^3$, mittl.Dichte 1,35 g/cm$^3$),der oben ein Kissen
von 30%iger Saccharose zur Absorption des g rößten Teils der
nicht-verwandten Proteine trug, gebandet, und die Fraktion mit einer Dichte von 1,34 g/cm$^3$ wurde durch Sedimentationszentrifugation über einen Saccharosegradienten gebandet.
HAV-Antigen-positive Fraktionen, getestet mit dem Standard-
Radioimmuntest der Feststoffphase, G.G. Frösner, Münch.
med. Wschr. 119 (1977) 825 unter Verwendung von HAVAB-
Test-Kits der Abbott Laboratories, North Chicago) wurden
zusammengegossen und dann erneut mit einem CsCl-Dichtegradienten ähnlich wie oben gebandet. Die Gesamtausbeute
von Partikeln aus dieser Zentrifugation band 10$^7$ cpm
von mit I$^{125}$ markiertem Anti-HAV. Dies entspricht ungefähr
einer Virusmasse von etwa 10 bis 20 ug.

## Synthese von komplementärer DNA (cDNA)

RNA wurde aus den Partikeln nach der Guanidin-Rhodanid-Methode
extrahiert (A. Ullrich, J. Shine, R. Chirgivin, E. Pictet,
W.J. Rutter und H.M. Goodman, Science 196 (1977) 1313),
so daß sichergestellt wurde, daß keine RNase-Aktivität
vorhanden war. Sie wurde mit 100 U reverser Transcriptase
des Myeloblastosis-Virus von Vögeln (Boehringer Mannheim) in Gegenwart von Oligo-d-$T_{12-18}$ als Primer, 8 mM $MgAc_2$, 0,01% NP-40®, Desoxyadenosintriphosphat (dATP), Desoxyguanidintriphosphat (dGTP), Desoxythymidintriphosphat (dTTP) je 1 mM, 0,2 mM Desoxycytidintriphosphat (dCTP) und 0,5 mCi $^{32}P$-dCTP als radioaktiver Nucleotidvorstufe (NEN) 2 Stunden bei 42°C transkribiert. Das gesamte Inkubationsgemisch wurde 1 Minute bei 80°C erhitzt und schnell gekühlt, worauf erneut zur Doppelstrang-Synthese 50 U reverse Transcriptase zugesetzt wurden und das Gemisch 2 Stunden bei 30°C inkubiert wurde. Die Analyse der komplementär-DNA (cDNA) durch Gelelektrophorese ergab eine ungefähre Ausbeute von 10 - 50 ng DNA einer uneinheitlichen Größe zwischen einigen Hundert bis zu 2000 Basenpaaren, wobei die größere Population bei etwa 800 bis 1000 Basenpaaren lag.

## Klonieren von cDNA in Plasmide

An die DNA wurde das Desoxycytidin am 3'-Ende (T. Nelson und D. Brutlag, Methods of Enzymology 68 (1979) 41, herausgegeben von R. Wu (Academic Pres New York)) unter Verwendung von terminaler Transferade (P.-L. Biochemicals Inc., Milwaukee) angehängt und dann mit Plasmid-pBR 322-DNA hybridisiert, die mit den Restriktionsenzymen PstI durchschnitten worden war, und an deren 3'-Enden Desoxyguanidin (dG)-Homopolymere angehängt worden waren (Nelson und Brutlag, loc. cit.). E. coli X 1776 wurde dann mit den Hybrid-Plasmiden transformiert und Klone wurden in L-Nährmedium in Gegenwart von Tetracyclin (12 µg/cm³) selektiert (H.M. Goodman und R.J. MacDonalt, Methods of Enzymology 68 (1979) 75, Herausgeber R. Wu (Academic Press, New York ).

## Analyse von Plasmid-Einfügungen

Einzelne bakterielle Klone wurden bis zu 100 ml in Gegenwart von Uridin gemäss M.V. Norgard, K. Emingholz und J. Monahan (J.Bact. 138 (1979), 270) gezüchtet und Plasmide wurden durch Lysis mit Triton X-100® und an-

schließende CsCl-Sedimentationszentrifugation in Gegenwart von Ethidiumbromid hergestellt (Villa-Komaroff, L., A. Efstradiadis, S. Broome, P. Lomedico, R. Tizard, S.P. Naber, W.L. Chick und W. Gilbert, Proc. Natl. Acad. Sci. USA 75 (1978) 3727 und F. Bolivar und K. Backman, Methods of Enzymology 68 (1979) 245, Herausgeber R. Wu (Academic Press, New York). Die Größe der Plasmide wurde durch Elektrophorese an 1%igem Agarosegel analysiert, und die Banden wurden mit Plasmid-Markern mit DNA-Einfügungungen von bekannter Größe verglichen.

## Hybridisierung von HAV-RNA zu Plasmid-DNA

Einzelne Bakterienklone wurden auf Nitrocellulose-Filtern über Agar gemäß M. Grundstein und D.S. Hogness (Proc. Natl. Acad. Sci. USA 72 (1975) 3961) gezüchtet. HAV-spezifische RNA wurde von den mit HAV infizierten PLC/PRF5-Zellen gewonnen (J. Alexander, G. Macnab, R. Saunders, Perspekctives in Virology 10 (1978) 103 (Herausgeber M. Pollard (Raven Press, New York). Vier Wochen nach der Infektion mit HAV (Frösner et al, 1979, loc.cit.; Deinhardt et al, 1981, loc.cit.), wurde den Zellen das Phosphat für 12 Std. entzogen, worauf sie mit $^{32}PO_4$ (NEN; 1 ci/cm³, trägerfrei( 24 Std. lang markiert und lysiert wurden. Die HAV-Partikel wurden durch CsCl-Sedimentationszentrifugation gereinigt. Eine Bande mit der Dichte von HAV (1,34 g/cm³), die bei einem HAV-Radioimmuntest eine positive Reaktion zeigte, wurde mit Phenol behandelt, und die RNA (100 000 cpm/Filter) wurde in 50%igem Formamid, 5 x SSC bei 40°C für 15 Stunden zu den auf Nitrocellulosefiltern gezüchteten Bakterienklonen hybridisiert. Jedes Filter enthielt einen negativen Kontroll-Klon mit dem Plasmid pBR 322, der keine Einfügung hatte.

-12-

## Sequenzieren der Hybrid-DNA

Die Hybrid-DNA wird mit der Endodesoxiribonucelase Pst I
aus dem Plasmidvektor ausgeschnitten und deren Nucleotidsequenz ermittelt nach den Methoden von A.M. Maxam, Walter
Gilbert (Proc.Natl.Acad.Sci. USA 74, 560, (1977)) oder
F. Sanger, S. Nicklen und A.R. Coulson (Proc.Natl.Acad.
Sci. USA 74, 5463 (1977)). Es werden so viele verschiedene
überlappende Klone sequestiert, bis die fortlaufende Nucleotidsequenz des HVA-Genoms feststeht.

## Expression von HAV-Antigen

Einzelne Bakterienklone wurden in jeweils 250 ml Suspension bis zu einer optischen Dichte (O.D.) von 0,8 gezüchtet
tet, worauf aliquote Teile von je 2, 20 und 200 ml zentrifugiert wurden. Die Bakterien-Pellets wurden mit Wasser
gemäß Villa-Komaroff et al (1978, loc.cit.) lysiert, und
aliquote Teile von 300 $\mu$l jedes Wasserschockfluids wurden
auf HAV-Antigene  in einem Festphasen-HAV-Radioimmuntest
(siehe oben) untersucht. Als Kontrolle wurden die beim
Radioimmuntest verwendeten Polystyrolkugeln mit Anti-HAV
Negativserum umhüllt.

## Anti-HAV-Peptide und deren Antikörper

Anhand der Nucleotidsequenz des HAV-Genoms wird die Aminosäuresequenz der HAV-Antigene errechnet und von den antigenen Epitopen Oligopeptide mit einer Länge von 9 bis 15 Aminosäuren synthetisiert. Diese Oligopeptide werden an nicht
immunogene Trägerproteine (wie KLH) gekoppelt und diese
Komplexe zur direkten Immunisierung verwendet oder um damit
im Versuchstier Antikörper gegen die HAV-Antigene herzustellen.

Man kann auch die HAV-spezifische Hybrid-DNA zur Darstellung von künstlichen Mutationen verwenden (die im RNA-Genom aufgrund biochemischer
Eigenschaften der RNA nicht durchzuführen sind), die einer natürlichen
Attenuierung gleichkommen, zur Herstellung von abgeschwächten Antigenen.

Verfahren zur Herstellung einer Hybrid-DNA, die das für das Hepatitis-A-Virus charakteristische komplementäre Genom enthält, dadurch gekennzeichnet, daß man doppelsträngige DNA, die durch reverse Transkription der viralen RNA des Hepatitis-A-Virus hergestellt worden ist, in einem Bakteriumplasmid kloniert.

Verfahren nach Anspuch    dadurch gekennzeichnet, daß die doppelsträngige DNA aus der ursprünglichen RNA des Hepatitis-A-Virus hergestellt worden ist, die mittels einer reversen Polymerase und den entsprechenden Vorläufer-Nukleotiden vorher  prepariert worden ist.

Verfahren nach Ansprüchen        ladurch gekennzeichnet, daß ein Escherichia coli-Plasmid, das die doppelsträngige DNA enthält, zur Klonierung verwendet wird.

Verfahren nach Ansprüchen      dadurch gekennzeichnet, daß die klonierten Plasmidvektoren mit einer Restriktionsendonuklease gespalten werden und die doppelsträngige DNA, die der DNA des Hepatitis-A-Virus entspricht, abgetrennt wird.

Plasmidvektor, dadurch gekennzeichnet, daß sein Genom die Hybrid-DNA nach Ansprüchen      enthält.

In Bakterienklone exprimierte Proteine oder Hybrid-Proteine, dadurch gekennzeichnet, daß sie HAV-spezifische Antigene enthalten, und von der Hybrid-DNA nach Anspruch codiert werden.

Verwendung der Hybrid-DNA nach Ansprüchen      bzw. des Plasmidvektors nach Anspruch 5 zum Nachweis von Hepatitis-A-Virus mittels molekularer Hybridisierung.

Verwendung der HAV-spezifischen Proteine nach Anspruch
zu diagnostischen Zwecken und zur Herstellung von Impfstoffen.

Aktiver Impfstoff gegen Hepatitis-A-Virus, dargestellt durch
Herstellung einer Hybrid-DNA nach Ansprüchen        kloniert
im Plasmidvektor nach Anspruch    exprimiert als die
Proteine oder Hybrid-Proteine nach Anspruch  , zusammen
mit üblichen Träger- und Hilfsstoffen.

Impfstoff gegen Hepatitis-A-Virus, enthaltend
Proteine oder Hybridproteine nach Anspruch 6

Passiver Impfstoff (Imunoglobuline) gegen Hepatitis-A-
Virus, dargestellt durch Immunisierung von Versuchstieren
mit Proteinen, die nach Ansprüchen        hergestellt
wurden.

Verwendung der HAV-spezifischen Hybrid-DNA nach Ansprüchen        zur Herstellung von künstlichen Mutationen,
die einer natürlichen Attenuierung gleichkommen.

Ansprüche
--------

1.      Verfahren zur Herstellung von Impfstoff, bei dem
Oligopeptide als Haptene plus Trägerproteine zur Immunisierung verwendet werden, wobei die Aminosäuresequenz dieser
Oligopeptide aus der Nucleotidsequenz der Hybrid-DNA nach
Ansprüchen 2 - 6 hergeleitet wird.


2.      Verfahren zur Herstellung einer Hybrid-DNA, die
das für das Hepatitis-A-Virus charakteristische komplementäre Genom enthält, dadurch gekennzeichnet, daß man doppelsträngige DNA, die durch reverse Transkription der viralen
RNA des Hepatitis-A-Virus hergestellt worden ist, in einem
Bakteriumplasmid kloniert.


3.      Verfahren nach Anspuch 2, dadurch gekennzeichnet, daß die doppelsträngige DNA aus der ursprünglichen
RNA des Hepatitis-A-Virus hergestellt worden ist, die
mittels einer reversen Polymerase und den entsprechenden Vorläufer-
Nukleotiden vorher  prepariert worden ist.


4.      Verfahren nach Ansprüchen 2 und 3, dadurch gekennzeichnet, daß ein Escherichia coli-Plasmid, das die
doppelsträngige DNA enthält, zur Klonierung verwendet
wird.


5.      Verfahren nach Ansprüchen 2 - 4, dadurch gekennzeichnet, daß die klonierten Plasmidvektoren mit einer
Restriktionsendonuklease gespalten werden und die doppelsträngige DNA, die der DNA des Hepatitis-A-Virus entspricht, abgetrennt wird.


6.      Plasmidvektor, dadurch gekennzeichnet, daß sein
Genom die Hybrid-DNA nach Ansprüchen 2 - 5 enthält.

$1/3$

plasmid
+ insertion
plasmid

a    b    c    d

Fig 1

*Fig. 2*

3/3

A    clone ⓪        ㉒        ㉙

B    clone ⓪        ㉒        ㉙

Fig 3.